# EUROPEAN PATENT APPLICATION

(11) **EP 2 218 458 A1**
(43) Date of publication of application: **18.08.2010**
(21) Application number: 09152778.8
(22) Date of filing: 13.02.2009
(51) Int. Cl.: A61K 38/17, C07K 14/47

(54) **Molecules able to modulate the expression of at least a gene involved in degradative pathways and uses thereof**

(71) Applicant: Fondazione Telethon, 00154 Roma (IT)
(72) Inventor: Ballabio, Andrea, 80131 Napoli (IT); Sardiello, Marco, 80131 Napoli (IT)
(74) Representative: Capasso, Olga

(57) **Abstract**

A molecule able to modulate the expression of at least a gene involved in degradative pathways so to enhance the cellular degradative pathways and prevent or antagonize the accumulation of toxic compounds in a cell wherein said molecule acts on a CLEAR element; and/or on the TFEB protein activity or amount.

The molecule may be used in the treatment of neurodegenerative and/or lysosomal storage disorders.

SEQUENCE LISTING

<110>  Fondazione Telethon

<120>  MOLECULES ABLE TO MODULATE THE EXPRESSION OF AT LEAST A GENE
       INVOLVED IN DEGRADATIVE PATHWAYS AND USES THEREOF

<130>  BE 105596

<160>  135

<170>  PatentIn version 3.5

<210>  1
<211>  22
<212>  DNA
<213>  Unknown

<220>
<223>  Synthetic primer

<400>  1
ccgcgcgtca cgtgacccca gc                                              22


<210>  2
<211>  22
<212>  DNA
<213>  Unknown

<220>
<223>  Synthetic primer

<400>  2
ccgcgccatt cgggacccca gc                                              22


<210>  3
<211>  14
<212>  DNA
<213>  Unknown

<220>
<223>  consensus CLEAR sequence

<400>  3
gggtcacgtg accc                                                       14


<210>  4
<211>  14
<212>  DNA
<213>  Unknown

<220>
<223>  CLEAR sequence

<400>  4
gcgtcgcgtg acgc                                                       14


<210>  5
<211>  14
<212>  DNA
<213>  Unknown

<220>
<223>  CLEAR sequence

<400>  5
agctcacctg gtgc                                                          14


<210>  6
<211>  14
<212>  DNA
<213>  Unknown

<220>
<223>  CLEAR sequence

<400>  6
ggctcacctg ggtc                                                          14


<210>  7
<211>  14
<212>  DNA
<213>  Unknown

<220>
<223>  CLEAR sequence

<400>  7
acctcaggtg atct                                                          14


<210>  8
<211>  14
<212>  DNA
<213>  Unknown

<220>
<223>  CLEAR sequence

<400>  8
gcgccaagtg actt                                                          14


<210>  9
<211>  14
<212>  DNA
<213>  Unknown

<220>
<223>  CLEAR sequence

<400>  9
gcgtcagctg agtt                                                          14


<210>  10
<211>  14
<212>  DNA
<213>  Unknown

<220>
<223>  CLEAR sequence

<400>  10
gagccacgtg tgcc                                                          14


<210>  11
<211>  14
<212>  DNA
<213>  Unknown

<220>
<223>  CLEAR sequence

<400>  11
gggtcacgcg gcgg                                                         14


<210>  12
<211>  14
<212>  DNA
<213>  Unknown

<220>
<223>  CLEAR sequence

<400>  12
acgtcacgtg ccgg                                                         14


<210>  13
<211>  14
<212>  DNA
<213>  Unknown

<220>
<223>  CLEAR sequence

<400>  13
gtgtcacgtg aggc                                                         14


<210>  14
<211>  14
<212>  DNA
<213>  Unknown

<220>
<223>  CLEAR sequence

<400>  14
ggaccacgtg gggt                                                         14


<210>  15
<211>  14
<212>  DNA
<213>  Unknown

<220>
<223>  CLEAR sequence

<400>  15
gggtctcgtg acgt                                                         14


<210>  16
<211>  14
<212>  DNA
<213>  Unknown

<220>
<223>  CLEAR sequence

<400>  16
cggtcacctg atct                                                         14


<210>  17
<211>  14

<212>  DNA
<213>  Unknown

<220>
<223>  CLEAR sequence

<400>  17
gggtcacgtg gtac                                                          14


<210>  18
<211>  14
<212>  DNA
<213>  Unknown

<220>
<223>  CLEAR sequence

<400>  18
gcatcacgtg ggcg                                                          14


<210>  19
<211>  14
<212>  DNA
<213>  Unknown

<220>
<223>  CLEAR sequence

<400>  19
aggtcatgtg acgc                                                          14


<210>  20
<211>  14
<212>  DNA
<213>  Unknown

<220>
<223>  CLEAR sequence

<400>  20
tggtcacgtg aggc                                                          14


<210>  21
<211>  14
<212>  DNA
<213>  Unknown

<220>
<223>  CLEAR sequence

<400>  21
gcgtcacctg acgc                                                          14


<210>  22
<211>  14
<212>  DNA
<213>  Unknown

<220>
<223>  CLEAR sequence

<400>  22
gcgtcagctg acct                                                          14

<210>  23
<211>  14
<212>  DNA
<213>  Unknown

<220>
<223>  CLEAR sequence

<400>  23
gggtcacgtg gggg                                                          14


<210>  24
<211>  14
<212>  DNA
<213>  Unknown

<220>
<223>  CLEAR sequence

<400>  24
gcatcacgtg ggga                                                          14


<210>  25
<211>  14
<212>  DNA
<213>  Unknown

<220>
<223>  CLEAR sequence

<400>  25
acgtcatgtg actg                                                          14


<210>  26
<211>  14
<212>  DNA
<213>  Unknown

<220>
<223>  CLEAR sequence

<400>  26
cggtcagctg actg                                                          14


<210>  27
<211>  14
<212>  DNA
<213>  Unknown

<220>
<223>  Synthetic primer

<400>  27
ctggcacgtg actt                                                          14


<210>  28
<211>  14
<212>  DNA
<213>  Unknown

<220>
<223>  CLEAR sequence

<400>  28
gggtcaagtg acac                                                          14


<210>  29
<211>  14
<212>  DNA
<213>  Unknown

<220>
<223>  CLEAR sequence

<400>  29
tggtcacctg agac                                                          14


<210>  30
<211>  14
<212>  DNA
<213>  Unknown

<220>
<223>  CLEAR sequence

<400>  30
cagtcacgtg cctc                                                          14


<210>  31
<211>  14
<212>  DNA
<213>  Unknown

<220>
<223>  CLEAR sequence

<400>  31
cgatcacgtg accg                                                          14


<210>  32
<211>  14
<212>  DNA
<213>  Unknown

<220>
<223>  CLEAR sequence

<400>  32
acctcacgtg acac                                                          14


<210>  33
<211>  14
<212>  DNA
<213>  Unknown

<220>
<223>  CLEAR sequence

<400>  33
cggtcacgtg acat                                                          14


<210>  34
<211>  14
<212>  DNA
<213>  Unknown

<220>
<223>  Synthetic primer

<400>  34
gggtcacgtg acgc                                                          14


<210>  35
<211>  14
<212>  DNA
<213>  Unknown

<220>
<223>  CLEAR SEQUENCE

<400>  35
ctctcacgtg accc                                                          14


<210>  36
<211>  14
<212>  DNA
<213>  Unknown

<220>
<223>  CLEAR sequence

<400>  36
gcgtcacatg aggg                                                          14


<210>  37
<211>  14
<212>  DNA
<213>  Unknown

<220>
<223>  CLEAR sequence

<400>  37
ggatcacttg aggc                                                          14


<210>  38
<211>  14
<212>  DNA
<213>  Unknown

<220>
<223>  CLEAR sequence

<400>  38
ggatcacctg aggt                                                          14


<210>  39
<211>  14
<212>  DNA
<213>  Unknown

<220>
<223>  CLEAR sequence

<400>  39
gattcaagtg attc                                                          14


<210>  40
<211>  14

<212>  DNA
<213>  Unknown

<220>
<223>  CLEAR sequence

<400>  40
acctcaagtg atcc                                                         14


<210>  41
<211>  14
<212>  DNA
<213>  Unknown

<220>
<223>  CLEAR sequence

<400>  41
tcatcacgtg gccc                                                         14


<210>  42
<211>  14
<212>  DNA
<213>  Unknown

<220>
<223>  CLEAR sequence

<400>  42
gcgtcacgtg gccg                                                         14


<210>  43
<211>  14
<212>  DNA
<213>  Unknown

<220>
<223>  CLEAR sequence

<400>  43
tgagcacgtg atgg                                                         14


<210>  44
<211>  14
<212>  DNA
<213>  Unknown

<220>
<223>  CLEAR sequence

<400>  44
ctgtcacgtg atcc                                                         14


<210>  45
<211>  14
<212>  DNA
<213>  Unknown

<220>
<223>  CLEAR sequence

<400>  45
gagtcacgtg aaca                                                         14

<210>  46
<211>  14
<212>  DNA
<213>  Unknown

<220>
<223>  CLEAR sequence

<400>  46
gggtcatgtg agcc                                                          14


<210>  47
<211>  14
<212>  DNA
<213>  Unknown

<220>
<223>  CLEAR sequence

<400>  47
gcgtcacgtg accc                                                          14


<210>  48
<211>  14
<212>  DNA
<213>  Unknown

<220>
<223>  CLEAR sequence

<400>  48
gagtcaggtg gcgg                                                          14


<210>  49
<211>  14
<212>  DNA
<213>  Unknown

<220>
<223>  CLEAR sequence

<400>  49
aggtcaggtg acag                                                          14


<210>  50
<211>  14
<212>  DNA
<213>  Unknown

<220>
<223>  CLEAR sequence

<400>  50
ctgtcacgtg gcgc                                                          14


<210>  51
<211>  14
<212>  DNA
<213>  Unknown

<220>
<223>  CLEAR sequence

<400>  51
agttcacgtg actc                                                          14


<210>  52
<211>  14
<212>  DNA
<213>  Unknown

<220>
<223>  CLEAR sequence

<400>  52
tggtcacgtg gggc                                                          14


<210>  53
<211>  14
<212>  DNA
<213>  Unknown

<220>
<223>  CLEAR sequence

<400>  53
ctttcacctg accc                                                          14


<210>  54
<211>  14
<212>  DNA
<213>  Unknown

<220>
<223>  CLEAR sequence

<400>  54
cgcccacgtg accg                                                          14


<210>  55
<211>  14
<212>  DNA
<213>  Unknown

<220>
<223>  CLEAR sequence

<400>  55
gggtcagctg atcc                                                          14


<210>  56
<211>  14
<212>  DNA
<213>  Unknown

<220>
<223>  CLEAR sequence

<400>  56
gccccacgtg cctc                                                          14


<210>  57
<211>  14
<212>  DNA
<213>  Unknown

<220>
<223>  CLEAR sequence

<400>  57
gaggcacttg aacc                                                          14


<210>  58
<211>  14
<212>  DNA
<213>  Unknown

<220>
<223>  CLEAR sequence

<400>  58
gcctcaagtg atcc                                                          14


<210>  59
<211>  14
<212>  DNA
<213>  Unknown

<220>
<223>  CLEAR sequence

<400>  59
agttcaggtg cccc                                                          14


<210>  60
<211>  14
<212>  DNA
<213>  Unknown

<220>
<223>  CLEAR sequence

<400>  60
gagtcacgtg cccc                                                          14


<210>  61
<211>  14
<212>  DNA
<213>  Unknown

<220>
<223>  CLEAR sequence

<400>  61
gcgtcacgtg accc                                                          14


<210>  62
<211>  14
<212>  DNA
<213>  Unknown

<220>
<223>  CLEAR sequence

<400>  62
cggtcacgtg accc                                                          14


<210>  63
<211>  14

<212>  DNA
<213>  Unknown

<220>
<223>  CLEAR sequence

<400>  63
gagtcatgtg accc                                                         14


<210>  64
<211>  14
<212>  DNA
<213>  Unknown

<220>
<223>  CLEAR sequence

<400>  64
gcgtcacgtg accc                                                         14


<210>  65
<211>  14
<212>  DNA
<213>  Unknown

<220>
<223>  CLEAR sequence

<400>  65
gagtcacgtg gccg                                                         14


<210>  66
<211>  14
<212>  DNA
<213>  Unknown

<220>
<223>  CLEAR sequence

<400>  66
gtgtcatgtg acgc                                                         14


<210>  67
<211>  14
<212>  DNA
<213>  Unknown

<220>
<223>  CLEAR sequence

<400>  67
tcatcacatg accc                                                         14


<210>  68
<211>  14
<212>  DNA
<213>  Unknown

<220>
<223>  CLEAR sequence

<400>  68
taatcacgtg agcg                                                         14

<210>  69
<211>  14
<212>  DNA
<213>  Unknown

<220>
<223>  CLEAR sequence

<400>  69
cggtcacgtg agca                                                          14


<210>  70
<211>  14
<212>  DNA
<213>  Unknown

<220>
<223>  CLEAR sequence

<400>  70
gagtcacgcg gcgg                                                          14


<210>  71
<211>  14
<212>  DNA
<213>  Unknown

<220>
<223>  CLEAR sequence

<400>  71
tagtcaagtg acgc                                                          14


<210>  72
<211>  14
<212>  DNA
<213>  Unknown

<220>
<223>  CLEAR sequence

<400>  72
ggctcaagtg atcc                                                          14


<210>  73
<211>  14
<212>  DNA
<213>  Unknown

<220>
<223>  CLEAR sequence

<400>  73
tggtcacgtg accg                                                          14


<210>  74
<211>  14
<212>  DNA
<213>  Unknown

<220>
<223>  CLEAR sequence

<400>  74
acttcacgtg accg                                                         14


<210>  75
<211>  14
<212>  DNA
<213>  Unknown

<220>
<223>  CLEAR sequence

<400>  75
ccgtcacgtg accg                                                         14


<210>  76
<211>  14
<212>  DNA
<213>  Unknown

<220>
<223>  CLEAR sequence

<400>  76
ggctcacgtg atcg                                                         14


<210>  77
<211>  14
<212>  DNA
<213>  Unknown

<220>
<223>  CLEAR sequence

<400>  77
acgtcacatg attc                                                         14


<210>  78
<211>  14
<212>  DNA
<213>  Unknown

<220>
<223>  CLEAR sequence

<400>  78
caatcacatg atcc                                                         14


<210>  79
<211>  14
<212>  DNA
<213>  Unknown

<220>
<223>  CLEAR sequence

<400>  79
gcgtcacgcg accc                                                         14


<210>  80
<211>  14
<212>  DNA
<213>  Unknown

<220>
<223>  CLEAR sequence

<400>  80
tggtcacgtg attc                                                          14


<210>  81
<211>  14
<212>  DNA
<213>  Unknown

<220>
<223>  CLEAR sequence

<400>  81
cgctcacctg acca                                                          14


<210>  82
<211>  14
<212>  DNA
<213>  Unknown

<220>
<223>  CLEAR sequence

<400>  82
gtctcacgtg gcca                                                          14


<210>  83
<211>  14
<212>  DNA
<213>  Unknown

<220>
<223>  CLEAR sequence

<400>  83
cagtcatctg actc                                                          14


<210>  84
<211>  14
<212>  DNA
<213>  Unknown

<220>
<223>  CLEAR sequence

<400>  84
cggtcacgtg cgcg                                                          14


<210>  85
<211>  14
<212>  DNA
<213>  Unknown

<220>
<223>  CLEAR sequence

<400>  85
cagccaggtg agcc                                                          14


<210>  86
<211>  14

<212>  DNA
<213>  Unknown

<220>
<223>  CLEAR sequence

<400>  86
ctgtcacctg gcgc                                                         14


<210>  87
<211>  14
<212>  DNA
<213>  Unknown

<220>
<223>  CLEAR sequence

<400>  87
ggctcacgtg cctg                                                         14


<210>  88
<211>  14
<212>  DNA
<213>  Unknown

<220>
<223>  CLEAR sequence

<400>  88
cgctcacgtg accc                                                         14


<210>  89
<211>  14
<212>  DNA
<213>  Unknown

<220>
<223>  CLEAR sequence

<400>  89
gggtcacctg aaca                                                         14


<210>  90
<211>  14
<212>  DNA
<213>  Unknown

<220>
<223>  CLEAR sequence

<400>  90
gagtcacgtg agcg                                                         14


<210>  91
<211>  14
<212>  DNA
<213>  Unknown

<220>
<223>  CLEAR sequence

<400>  91
ctgtcacgtg acgc                                                         14

<210>  92
<211>  14
<212>  DNA
<213>  Unknown

<220>
<223>  CLEAR sequence

<400>  92
gggtcacgtg ggtc                                                          14


<210>  93
<211>  14
<212>  DNA
<213>  Unknown

<220>
<223>  CLEAR sequence

<400>  93
gggtcacgtg ccgc                                                          14


<210>  94
<211>  14
<212>  DNA
<213>  Unknown

<220>
<223>  CLEAR sequence

<400>  94
gggtcacgtg ccgc                                                          14


<210>  95
<211>  14
<212>  DNA
<213>  Unknown

<220>
<223>  CLEAR sequence

<400>  95
gggtcacgtg tcgg                                                          14


<210>  96
<211>  14
<212>  DNA
<213>  Unknown

<220>
<223>  CLEAR sequence

<400>  96
ggatcacgtg acgc                                                          14


<210>  97
<211>  14
<212>  DNA
<213>  Unknown

<220>
<223>  CLEAR sequence

<400>  97
agctcacgtg acaa                                                          14


<210>  98
<211>  14
<212>  DNA
<213>  Unknown

<220>
<223>  CLEAR sequence

<400>  98
tggtcacatg ccgc                                                          14


<210>  99
<211>  14
<212>  DNA
<213>  Unknown

<220>
<223>  CLEAR sequence

<400>  99
cgctcagctg acct                                                          14


<210>  100
<211>  14
<212>  DNA
<213>  Unknown

<220>
<223>  CLEAR sequence

<400>  100
cggtcacgtg aggg                                                          14


<210>  101
<211>  14
<212>  DNA
<213>  Unknown

<220>
<223>  CLEAR sequence

<400>  101
gggtcacgtg accg                                                          14


<210>  102
<211>  14
<212>  DNA
<213>  Unknown

<220>
<223>  CLEAR sequence

<400>  102
agatcagctg atgc                                                          14


<210>  103
<211>  14
<212>  DNA
<213>  Unknown

<220>
<223>  CLEAR sequence

<400>  103
ccgtcaggtg cgca                                                         14


<210>  104
<211>  14
<212>  DNA
<213>  Unknown

<220>
<223>  CLEAR sequence

<400>  104
cggtcacgag acgc                                                         14


<210>  105
<211>  14
<212>  DNA
<213>  Unknown

<220>
<223>  CLEAR sequence

<400>  105
gagtcacgtg aggc                                                         14


<210>  106
<211>  14
<212>  DNA
<213>  Unknown

<220>
<223>  CLEAR sequence

<400>  106
agcccacatg accc                                                         14


<210>  107
<211>  14
<212>  DNA
<213>  Unknown

<220>
<223>  CLEAR sequence

<400>  107
gggtcacgcg ctcc                                                         14


<210>  108
<211>  14
<212>  DNA
<213>  Unknown

<220>
<223>  CLEAR sequence

<400>  108
gggtcagctg actc                                                         14


<210>  109
<211>  14

<212>  DNA
<213>  Unknown

<220>
<223>  CLEAR sequence

<400>  109
cagtcacgtg accc                                                          14


<210>  110
<211>  14
<212>  DNA
<213>  Unknown

<220>
<223>  CLEAR sequence

<400>  110
ccatcacgtg accg                                                          14


<210>  111
<211>  14
<212>  DNA
<213>  Unknown

<220>
<223>  CLEAR sequence

<400>  111
ccgtcacgtg accg                                                          14


<210>  112
<211>  14
<212>  DNA
<213>  Unknown

<220>
<223>  CLEAR sequence

<400>  112
cggtcatgtg acac                                                          14


<210>  113
<211>  14
<212>  DNA
<213>  Unknown

<220>
<223>  CLEAR sequence

<400>  113
ggatcagctg acgc                                                          14


<210>  114
<211>  14
<212>  DNA
<213>  Unknown

<220>
<223>  CLEAR sequence

<400>  114
aaatcaagtg accc                                                          14

<210>  115
<211>  14
<212>  DNA
<213>  Unknown

<220>
<223>  CLEAR sequence

<400>  115
ccgtcaggtg ggga                                                         14


<210>  116
<211>  14
<212>  DNA
<213>  Unknown

<220>
<223>  CLEAR sequence

<400>  116
cccgcacgtg aggg                                                         14


<210>  117
<211>  14
<212>  DNA
<213>  Unknown

<220>
<223>  CLEAR sequence

<400>  117
gcctcagatg aggc                                                         14


<210>  118
<211>  14
<212>  DNA
<213>  Unknown

<220>
<223>  CLEAR sequence

<400>  118
ggggcaggtg aggc                                                         14


<210>  119
<211>  14
<212>  DNA
<213>  Unknown

<220>
<223>  CLEAR sequence

<400>  119
ccgtcacgtg atgg                                                         14


<210>  120
<211>  14
<212>  DNA
<213>  Unknown

<220>
<223>  CLEAR sequence

<400>  120
cgcgcacgtg accg                                                          14


<210>  121
<211>  14
<212>  DNA
<213>  Unknown

<220>
<223>  CLEAR sequence

<400>  121
ggagcacgtg acct                                                          14


<210>  122
<211>  14
<212>  DNA
<213>  Unknown

<220>
<223>  CLEAR sequence

<400>  122
ggatcacgtg atga                                                          14


<210>  123
<211>  14
<212>  DNA
<213>  Unknown

<220>
<223>  CLEAR sequence

<400>  123
cagccacgtg acca                                                          14


<210>  124
<211>  14
<212>  DNA
<213>  Unknown

<220>
<223>  CLEAR sequence

<400>  124
gggtcacatg gccc                                                          14


<210>  125
<211>  14
<212>  DNA
<213>  Unknown

<220>
<223>  CLEAR sequence

<400>  125
gaaacacgtg acca                                                          14


<210>  126
<211>  14
<212>  DNA
<213>  Unknown

<220>
<223>  CLEAR sequence

<400>  126
aggtcatgtg acag                                                          14


<210>  127
<211>  14
<212>  DNA
<213>  Unknown

<220>
<223>  CLEAR sequence

<400>  127
gcgtcacgtg acgg                                                          14


<210>  128
<211>  14
<212>  DNA
<213>  Unknown

<220>
<223>  CLEAR sequence

<400>  128
agctcatgtg atcc                                                          14


<210>  129
<211>  14
<212>  DNA
<213>  Unknown

<220>
<223>  CLEAR sequence

<400>  129
ccgtcacatg acag                                                          14


<210>  130
<211>  14
<212>  DNA
<213>  Unknown

<220>
<223>  CLEAR sequence

<400>  130
taatcaggtg actc                                                          14


<210>  131
<211>  14
<212>  DNA
<213>  Unknown

<220>
<223>  CLEAR sequence

<400>  131
cgaccacgtg attc                                                          14


<210>  132
<211>  14

<212>  DNA
<213>  Unknown

<220>
<223>  CLEAR sequence

<400>  132
agctcacgtg acaa                                                         14


<210>  133
<211>  14
<212>  DNA
<213>  Unknown

<220>
<223>  CLEAR sequence

<400>  133
gagtcagctg agct                                                         14


<210>  134
<211>  14
<212>  DNA
<213>  Unknown

<220>
<223>  CLEAR sequence

<400>  134
ggatcacctg gcac                                                         14


<210>  135
<211>  14
<212>  DNA
<213>  Unknown

<220>
<223>  CLEAR sequence

<400>  135
acaccacgtg acga                                                         14

## Description

### Field of the invention

The invention refers to molecules able to modulate the expression of at least a gene involved in degradative pathways so to enhance the cellular degradative pathways and prevent or antagonize the accumulation of toxic compounds in a cell.

### Background of the invention

Lysosomal storage disorders and neurodegenerative diseases such as Alzheimer's, Parkinson's, and Huntington's share as a common feature the progressive accumulation of undigested macromolecules within the cell, either proteins that tend to form pathogenic aggregates, or intermediates of the cellular catabolism. This ultimately results in cellular dysfunction and clinical manifestations with variable association of visceral (hepatosplenomegaly), skeletal (joint limitation, bone disease and deformities), hematologic (anemia, lymphocyte vacuolization and inclusions), and, most importantly, neurological involvement, with often irreversible damage and invalidating or fatal consequences. Since all of these disorders share a reduced digestive capability of the cell, it would be of great medical interest to identify molecules able to act as general enhancers of degradative pathways.

Prior art reports the description of a system to increase the activity of some cathepsins following the inhibition of the lysosomal system; however, these results are rather partial, controversial, and the molecular mechanism has not been analyzed into details. In the published literature there are no papers that reveal the presence of a lysosomal gene network or that identify TFEB as a possible modulator of the lysosomal activity.

Patent application CA 2525255 A1 describes the use of TFEB for cancer treatment and for modulating cell proliferation or differentiation; patent application WO 2007/070856 A2 claims the use of TFEB for treating immune dysfunction. None of the two previous documents show nor suggest the use of TFEB to enhance the cellular degradation pathway. Moreover, the CLEAR regulatory element - allowing the lysosomal system modulation - is not disclosed in any prior art documents.

Technologies able to enhance the lysosomal activity have not been described so far. Authors defined molecular events involved in the modulation of the lysosomal system through the regulatory element CLEAR or the TFEB protein.

### Description of the invention

The authors of the invention identified a gene network that comprises the genes encoding lysosomal proteins of critical importance for the degradation of toxic compounds. These proteins are involved, directly or indirectly, in a high number of human diseases. The regulatory element responsible for the modulation of these genes has been identified in their promoter sequences. Such regulatory element, which authors called CLEAR, represents itself a target for the modulation - and therefore the enhancement - of the production of the lysosomal proteins responsible for the degradation of toxic compounds. Finally, a transcription factor, called TFEB, (NCBI GeneID = 7942; RefSeq IDs, nt = NM_007162.1, protein = NP_009093.1) has been identified as a protein able to bind to the CLEAR element and to modulate the expression of target genes. Authors demonstrated that the lysosomal activity can be modulated by increasing or decreasing the amount of TFEB. In particular, the lysosomal enhancement resulting from the increase in TFEB levels is able to clear the cell from the toxic protein responsible for the neurodegenerative Huntington's disease.

The enhancement of the cellular degradative pathways by the activation of the lysosomal system may be advantageously used for the therapy of lysosomal storage disorders and neurodegenerative disease such as Alzheimer's disease, Parkinson's disease and Huntington's disease.

Such treatment may be performed by using:
1) molecules, as peptides or any other chemicals, able to act directly on the CLEAR element, responsible for the modulation of the expression of lysosomal genes and other genes involved in degradative pathways, in order to enhance the cellular degradative pathways and prevent or antagonize the accumulation of toxic compounds; and/or
2) molecules, as peptides, microRNAs, microRNA inhibitors, or any other chemicals, able to act directly or indirectly on the TFEB protein or on its amount, being the TFEB protein able to enhance the cellular degradative pathways by binding to the regulatory element CLEAR; and/or
3) vectors for gene therapy containing TFEB, microRNAs, microRNA inhibitors, or other genes able to modulate the CLEAR regulatory network, in order to enhance the cellular degradative pathways.

In the instant invention, lysosomal storage disorders are intended as inherited diseases in which a defect in one of many proteins participating in lysosomal biogenesis or metabolism leads to the intralysosomal storage of undegraded molecules, as described in "Lysosomes", author: Paul Saftig, Landes Bioscience, 2005.

It is a specific object of the invention a molecule able to modulate the expression of at least a gene involved in degradative pathways so to enhance the cellular degradative pathways and prevent or antagonize the accumulation of toxic compounds in a cell wherein said molecule acts either on a CLEAR element comprising at least one repeat of a nucleotide sequence having SEQ ID N 3 as consensus sequence; or on the TFEB protein activity or amount. The expert in the field shall select the CLEAR sequence by available programs. As preferred embodiments the molecule acts on a CLEAR element comprising at least one repeat of a nucleotide sequence selected from the group from SEQ ID N. 3 to SEQ ID N. 135. Such CLEAR elements were identified according to the Positional weight matrix (PWM) as described below and in Table 1. The expert may easily and with no further inventive effort identify other sequences that fulfil the criteria ans that therefore are within the scope of the instant invention. Moreover the expert may easily end with no further inventive effort construct repeats including same or different CLEAR sequences. Preferably repeats are in a number comprised between two and ten.

Preferably the gene involved in degradative pathways is a gene expressing a lysosomal protein.

Preferably the molecule is a peptide.

More preferably the molecule is the TFEB protein or a functional fragment thereof or a mutein thereof, namely a mutated TFEB protein or functional fragment thereof that is able to enhance the expression of a gene involved in degradative pathways. The expert in the field easily may identify the sequence of such fragments or muteins by transfecting appropriate recombinant expressing vectors in cells or tissues and subsequently monitoring the changes in the expression of genes involved in degradative pathways. Many techniques are known to the expert as, i.e, real-time quantitative PCR, expression microarray analysis, or analogous techniques.

In an alternative embodiment the molecule is a microRNA or microRNA inhibitor.

It is a preferred embodiment of the invention the molecule for medical use, particularly for neurodegenerative disorders' treatments, as i.e. Alzheimer, Parkinson and Huntington diseases; alternatively for lysosomal storage disorders' treatments.

It is another aspect of the invention a nucleic acid containing a coding sequence encoding for the molecule as above described, and a vector for gene therapy comprising under appropriate regulative sequence the same.

The invention shall be described with reference to experimental non limitating evidences.

### Description of figures

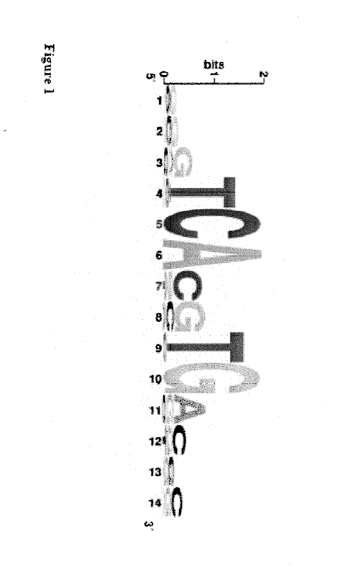

- Figure 1. Logo representation of the Coordinated Lysosomal Expression And Regulation (CLEAR) element. The overall height of each column is proportional to the information content at that position, and within columns the conservation of each residue is visualized as the relative height of symbols representing nucleotides.

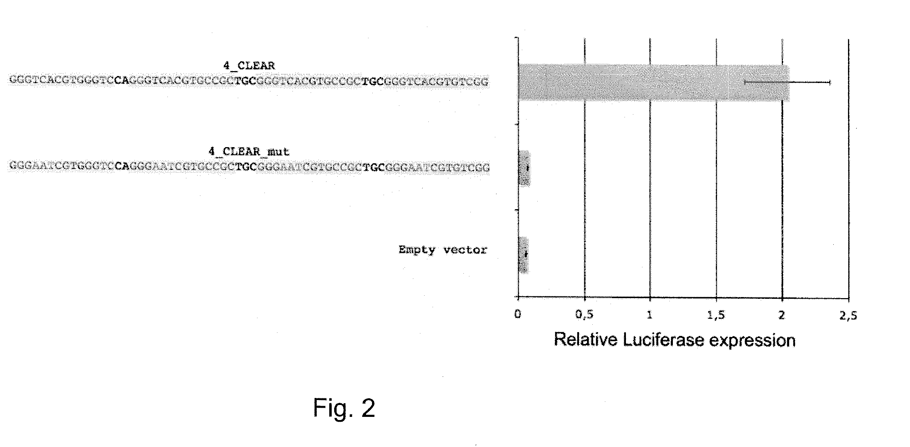

- Figure 2 . Luciferase assay demonstrating that CLEAR sites promote transcription. Two different luciferase constructs were prepared with the pGL3 system (Promega): (i) 4_CLEAR, carrying four tandem copies of the CLEAR element upstream of the luciferase reporter gene (CLEAR sites are shaded in orange); (ii) 4_CLEAR_mut, carrying mutations in each of the four CLEAR elements which destroy the CLEAR consensi (mutations are indicated in red). 4_CLEAR, 4_CLEAR_mut, or empty pGL3 vectors were co-transfected in cultured HeLa cells with a renilla reporter vector to normalize luciferase measurement. The results show that the presence of intact CLEAR sites increase luciferase expression compared to mutated sites or to empty pGL3 vector. The 4_CLEAR sequence reproduces exactly a portion of the human LAMP1 promoter (from -111 to -47 relative to the transcription start site). The essay was performed following manufacturer's instructions.

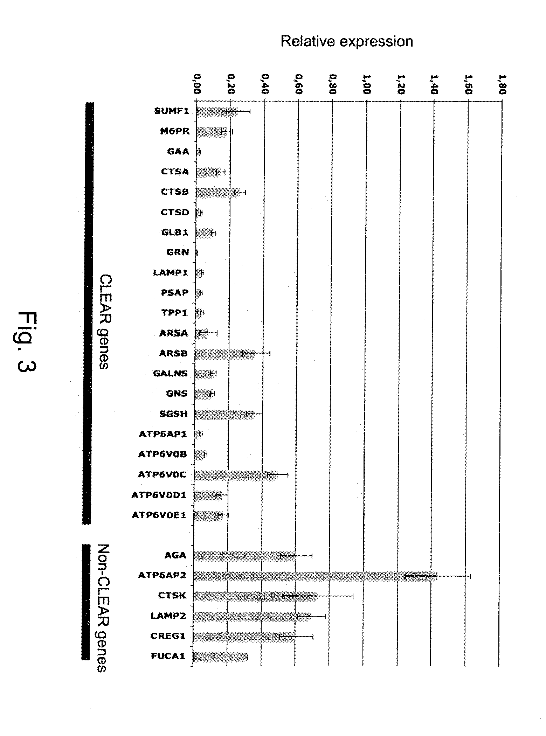

- Figure 3 . DNA decoy experiments. Cultured HeLa cells were transfected with 0.5ug of double strand oligonucleotides carrying a CLEAR site (or a scrambled sequence in the control) to sequester the cognate transcription factor from the lysosomal promoters bound in vivo. Real-time qPCR was performed to monitor the expression of a representative pool of lysosomal genes 48 hours after transfection. The graph shows the ratio between the expression levels of lysosomal genes in CLEAR- vs. scramble-transfected cells. The results show a general reduction in the expression of CLEAR genes, with a dramatic silencing (>80%) in most cases. Conversely, the expression of several lysosomal non-CLEAR genes appears scarcely affected. Gene expression was normalized relative to GAPDH. DNA transfections were performed using the following oligos:
   CLEAR, 5'-CCGCGCGTCACGTGACCCCAGC-3' (SEQ ID No. 1) and complementary sequence;
   scramble, 5'-CCGCGCCATTCGGGACCCCAGC-3' (SEQ ID No. 2) and complementary sequence.

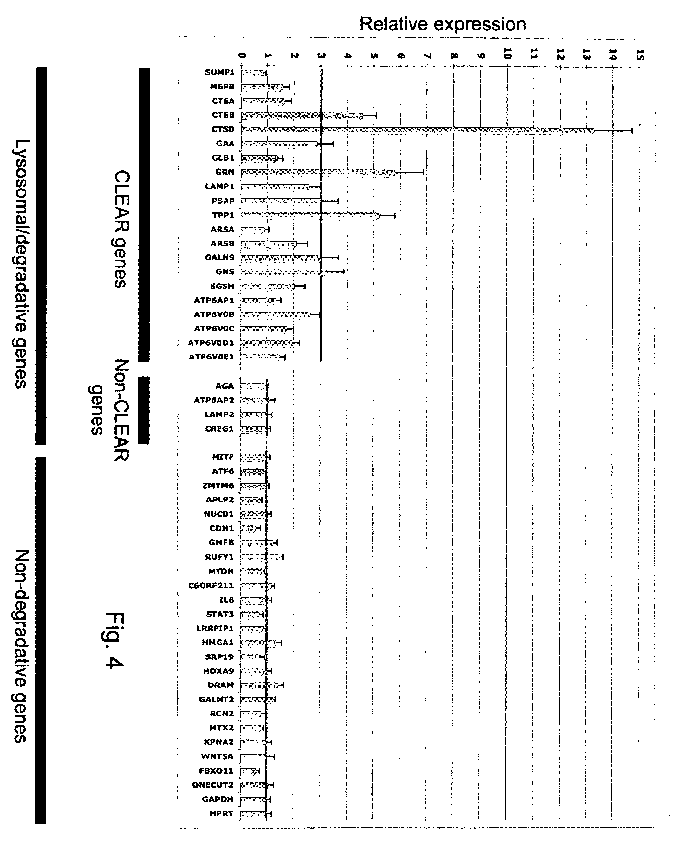

- Figure 4 . The over-expression of TFEB promotes the upregulation of CLEAR genes. Cultured HeLa cells were transfected with pcDNA3.1 (Invitrogen) carrying the human TFEB cDNA, or with empty pcDNA3.1 in the control. Real-time qPCR was performed to monitor the expression of a representative pool of lysosomal genes 24 hours after transfection. The graph shows the ratio between the expression levels of lysosomal genes in TFEB- vs. pcDNA3.1-transfected cells. The results show a general increase in the expression of CLEAR genes (average fold change = 3). Conversely, the expression of several monitored non-CLEAR lysosomal genes and random non-CLEAR genes was not affected (average fold change = 1). Gene expression was normalized relative to GAPDH. Average fold changes for each group of genes are represented as green bars.

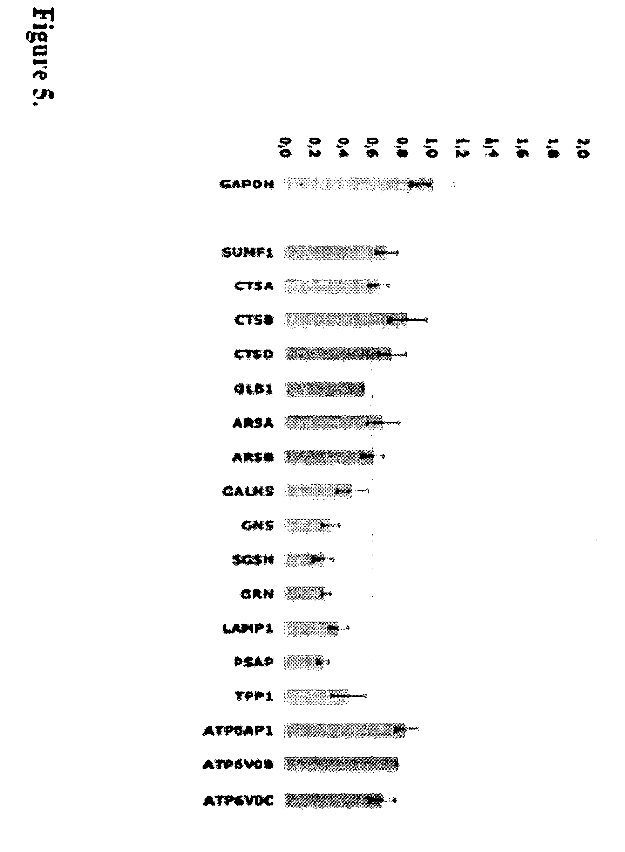

- Figure 5. Silencing of TFEB results in the down-regulation of CLEAR genes. Cultured HeLa cells were transfected with siRNAs specific to TFEB (iTFEB)(Dharmacon), or with a control siRNA (iCTRL) that has no targets in human cells. Real-time qPCR was performed to monitor the expression of a representative pool of CLEAR genes 48 hours after transfection. The graph shows the ratio between the expression levels of CLEAR genes in iTFEB- vs. iCTRL-transfected cells. The results show a general decrease in the expression of CLEAR genes (average fold change = 0.5). Gene expression was normalized relative to GAPDH.

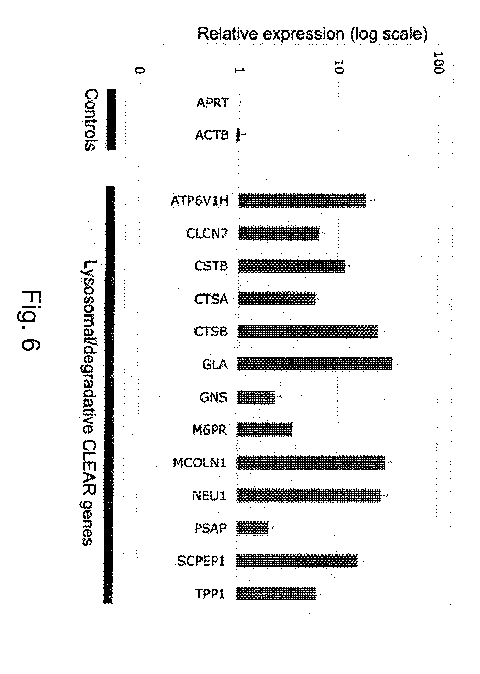

- Figure 6. TFEB directly binds to lysosomal promoters. HeLa cells were transfected with a plasmid carrying a 3xFLAG-TFEB cDNA and selected for stable expression of this transgene. Cell extracts were subjected to chromatin immunoprecipitation with an anti-FLAG antibody and purified for real-time qPCR analysis. The graph shows the ratio between the amount of several lysosomal promoters and the beta-actin promoter (control gene) in the immunoprecipitated fraction. The results show a significant enrichment of lysosomal promoters in the chromatin fraction bound by TFEB.

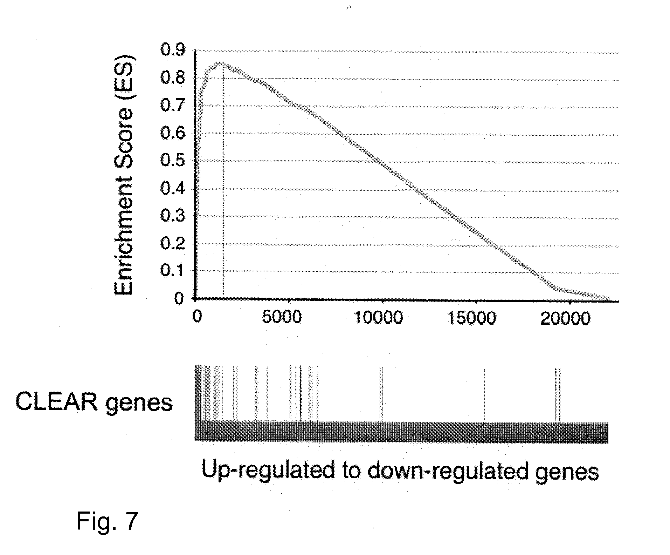

- Figure 7. Gene Set Enrichment Score Analysis (GSEA) of transcriptome changes following TFEB overexpression. The graph shows the enrichment plots generated by GSEA analysis of ranked gene expression data (left: upregulated, red; right: downregulated, blue). The enrichment score is shown as a blue line, and the vertical blue bars below the plot indicate the position of genes carrying CLEAR sites at their promoters. The analysis shows that CLEAR genes are mostly grouped in the fraction of up-regulated genes (Enrichment Score = 0.84; P<0.0001).

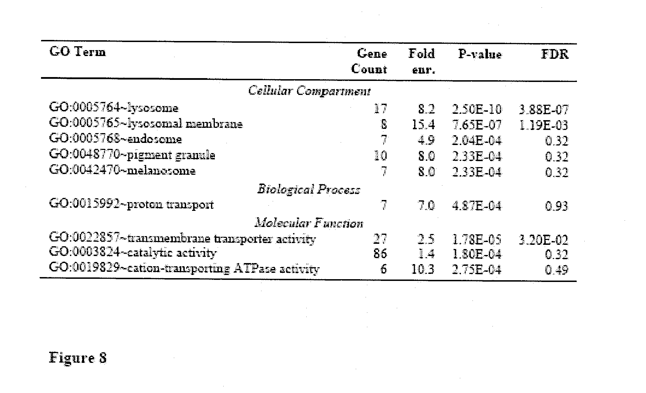

- Figure 8. Gene Ontology (GO) categories enriched within the set of genes upregulated following TFEB transient overexpression.

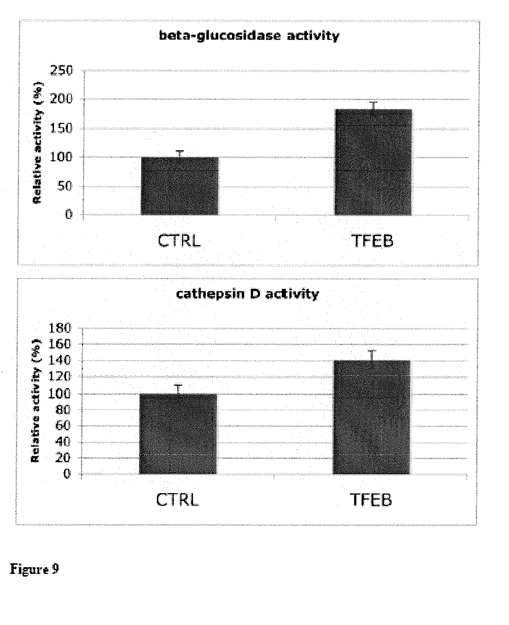

- Figure 9. Enzymatic assays to compare the activity of lysosomal enzymes beta-glucosidase and cathepsin D in TFEB-overexpressing HeLa (TFEB) versus control HeLa (CTRL). The results show that the overexpression of TFEB results in a significant increase in the activity of both hydrolases.

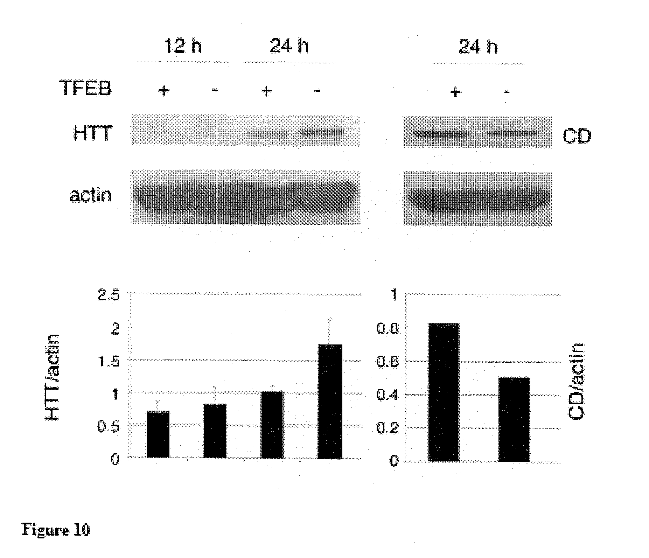

- Figure 10. TFEB promotes Huntingtin clearance. Western blot analysis of HD43 cells at 12 and 24 hours following HD43 induction, electroporated with a plasmid carrying the TFEB cDNA or an empty vector as a control. HTT, Huntingtin isoform HD43(Q105); CD, cathepsin D.

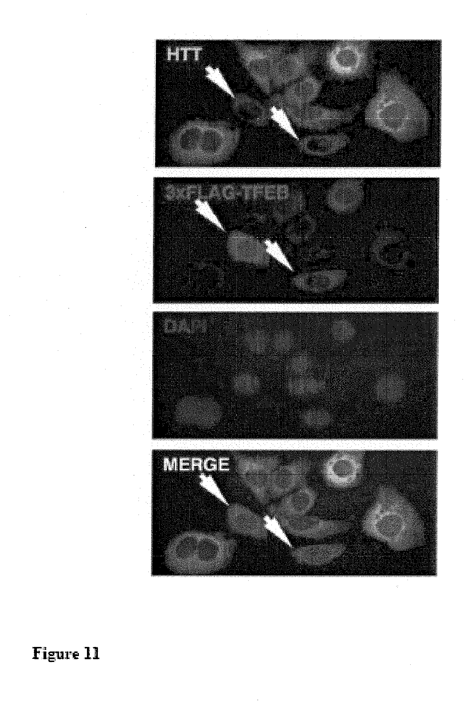

- Figure 11. Immunofluorescence analysis of HD43 cells at 24 hours following 3xFLAG-TFEB transfection. The arrows indicate cells over-expressing 3xFLAG-TFEB. The pictures show that cells over-expressing 3xFLAG-TFEB display a significantly minor content of Huntingtin (HTT) than non-transfected cells.

### Experimental evidences

By performing a sequence analysis of lysosomal gene promoters, authors found that most of them share a short sequence with structural features typically associated to regulatory sequences:
(i) the sequence consensus (GGGTCACGTGACCC, SEQ ID No. 3) is a perfect palindrome (Figure 1);
(ii) It is found within 200 bp from the transcription start site of lysosomal genes;
(iii) It is present as tandem repeats in a significant proportion of promoters;
(iv) It is conserved between human and mouse promoters.

To identify CLEAR sites in DNA sequences, the authors used the Positional Weight Matrix (PWM) reported in Table 1 (CLEAR PWM) and the web tool PATSER (available at http://rsat.scmbb.ulb.ac.be/rsat/) with the following parameters: Lower threshold estimation (weight score) = 4; Upper threshold = none; Alphabet = a:t 0.3 c:g 0.2 (Thomas-Chollier, M. et al. RSAT: regulatory sequence analysis tools. Nucleic Acids Res 36e, W119-27, 2008).

**Table 1 - Positional weight matrix (PWM) describing CLEAR sequences (CLEAR PWM).**

| | | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| A | 10 | 13 | 15 | 1 | 0 | 92 | 6 | 2 | 0 | 0 | 79 | 5 | 5 | 11 |
| C | 19 | 25 | 19 | 9 | 94 | 0 | 74 | 12 | 2 | 0 | 5 | 55 | 51 | 54 |
| G | 54 | 51 | 55 | 5 | 0 | 2 | 12 | 74 | 0 | 94 | 9 | 19 | 25 | 19 |
| T | 11 | 5 | 5 | 79 | 0 | 0 | 2 | 6 | 92 | 0 | 1 | 15 | 13 | 10 |

The occurrence of each nucleotide at each position is shown for a set of lysosomal CLEAR elements. The CLEAR PWM can be used to identify CLEAR elements in any given DNA sequences, by using the web tool PATSER as specified in the main text.

Table 2 show sequences of examples of CLEAR elements found in promoters of genes (left column) participating to degradative pathways.

**Table 2**

| **Gene name** | **CLEAR element sequence** | |
|---|---|---|
| ABCA2 | GCGTCGCGTGACGC | SEQ ID N4 |
| ABCB9 | AGCTCACCTGGTGC | SEQ ID N5 |
| ACP5 | GGCTCACCTGGGTC | SEQ ID N6 |
| ARL8B | ACCTCAGGTGATCT | SEQ ID N7 |
| ARSA | GCGCCAAGTGACTT | SEQ ID N8 |
| ARSB | GCGTCAGCTGAGTT | SEQ ID N9 |
| ARSG | GAGCCACGTGTGCC | SEQ ID N10 |
| ASAH1 | GGGTCACGCGGCGG | SEQ ID N11 |
| ATG3 | ACGTCACGTGCCGG | SEQ ID N12 |
| ATG3 | GTGTCACGTGAGGC | SEQ ID N13 |
| ATG5 | GGACCACGTGGGGT | SEQ ID N14 |
| ATG5 | GGGTCTCGTGACGT | SEQ ID N15 |
| ATP6AP1 | CGGTCACCTGATCT | SEQ ID N16 |
| ATP6VOB | GGGTCACGTGGTAC | SEQ ID N17 |
| ATP6VOB | GCATCACGTGGGCG | SEQ ID N18 |
| ATP6VOC | AGGTCATGTGACGC | SEQ ID N19 |
| ATP6V0D1 | TGGTCACGTGAGGC | SEQ ID N20 |
| ATP6V0D1 | GCGTCACCTGACGC | SEQ ID N21 |
| ATP6V0D1 | GCGTCAGCTGACCT | SEQ ID N22 |
| ATP6V0E1 | GGGTCACGTGGGGG | SEQ ID N23 |
| ATP6V0E2 | GCATCACGTGGGGA | SEQ ID N24 |
| ATP6V1A | ACGTCATGTGACTG | SEQ ID N25 |
| ATP6V1C1 | CGGTCAGCTGACTG | SEQ ID N26 |
| ATP6V1C1 | CTGGCACGTGACTT | SEQ ID N27 |
| ATP6V1D | GGGTCAAGTGACAC | SEQ ID N28 |
| ATP6V1E2 | TGGTCACCTGAGAC | SEQ ID N29 |
| ATP6V1H | CAGTCACGTGCCTC | SEQ ID N30 |
| ATP6V1H | CGATCACGTGACCG | SEQ ID N31 |
| BLOC1S1 | ACCTCACGTGACAC | SEQ ID N32 |
| BLOC1S1 | CGGTCACGTGACAT | SEQ ID N33 |
| C2orf18 | GGGTCACGTGACGC | SEQ ID N34 |
| CD164 | CTCTCACGTGACCC | SEQ ID N35 |
| CD63 | GCGTCACATGAGGG | SEQ ID N36 |
| CECR1 | GGATCACTTGAGGC | SEQ ID N37 |
| CECR1 | GGATCACCTGAGGT | SEQ ID N38 |
| CECR1 | GATTCAAGTGATTC | SEQ ID N39 |
| CECR1 | ACCTCAAGTGATCC | SEQ ID N40 |
| CLCN7 | TCATCACGTGGCCC | SEQ ID N41 |
| CLCN7 | GCGTCACGTGGCCG | SEQ ID N42 |
| CLN3 | TGAGCACGTGATGG | SEQ ID N43 |
| CLN3 | CTGTCACGTGATCC | SEQ ID N44 |
| CORO1B | GAGTCACGTGAACA | SEQ ID N45 |
| CPVL | GGGTCATGTGAGCC | SEQ ID N46 |
| CSTB | GCGTCACGTGACCC | SEQ ID N47 |
| CTNS | GAGTCAGGTGGCGG | SEQ ID N48 |
| CTNS | AGGTCAGGTGACAG | SEQ ID N49 |
| CTSA | CTGTCACGTGGCGC | SEQ ID N50 |
| CTSA | AGTTCACGTGACTC | SEQ ID N51 |
| CTSB | TGGTCACGTGGGGC | SEQ ID N52 |
| CTSC | CTTTCACCTGACCC | SEQ ID N53 |
| CTSD | CGCCCACGTGACCG | SEQ ID N54 |
| CTSD | GGGTCAGCTGATCC | SEQ ID N55 |
| CTSF | GCCCCACGTGCCTC | SEQ ID N56 |
| CTSO | GAGGCACTTGAACC | SEQ ID N57 |
| CTSS | GCCTCAAGTGATCC | SEQ ID N58 |
| CTSZ | AGTTCAGGTGCCCC | SEQ ID N59 |
| FCGRT | GAGTCACGTGCCCC | SEQ ID N60 |
| GAA | GCGTCACGTGACCC | SEQ ID N61 |
| GAA | CGGTCACGTGACCC | SEQ ID N62 |
| GALC | GAGTCATGTGACCC | SEQ ID N63 |
| GALNS | GCGTCACGTGACCC | SEQ ID N64 |
| GALNS | GAGTCACGTGGCCG | SEQ ID N65 |
| GBA | GTGTCATGTGACGC | SEQ ID N66 |
| GBA | TCATCACATGACCC | SEQ ID N67 |
| GLA | TAATCACGTGAGCG | SEQ ID N68 |
| GLA | CGGTCACGTGAGCA | SEQ ID N69 |
| GLB1 | GAGTCACGCGGCGG | SEQ ID N70 |
| GLB1 | TAGTCAAGTGACGC | SEQ ID N71 |
| GNPTAB | GGCTCAAGTGATCC | SEQ ID N72 |
| GNPTG | TGGTCACGTGACCG | SEQ ID N73 |
| GNPTG | ACTTCACGTGACCG | SEQ ID N74 |
| GNS | CCGTCACGTGACCG | SEQ ID N75 |
| GNS | GGCTCACGTGATCG | SEQ ID N76 |
| GRN | ACGTCACATGATTC | SEQ ID N77 |
| GRN | CAATCACATGATCC | SEQ ID N78 |
| GUSB | GCGTCACGCGACCC | SEQ ID N79 |
| HEXA | TGGTCACGTGATTC | SEQ ID N80 |
| HEXA | CGCTCACCTGACCA | SEQ ID N81 |
| HEXA | GTCTCACGTGGCCA | SEQ ID N82 |
| HEXB | CAGTCATCTGACTC | SEQ ID N83 |
| HPS1 | CGGTCACGTGCGCG | SEQ ID N84 |
| HPSE | CAGCCAGGTGAGCC | SEQ ID N85 |
| HYAL2 | CTGTCACCTGGCGC | SEQ ID N86 |
| IFI30 | GGCTCACGTGCCTG | SEQ ID N87 |
| IGF2R | CGCTCACGTGACCC | SEQ ID N88 |
| IGF2R | GGGTCACCTGAACA | SEQ ID N89 |
| IGF2R | GAGTCACGTGAGCG | SEQ ID N90 |
| IGF2R | CTGTCACGTGACGC | SEQ ID N91 |
| LAMP1 | GGGTCACGTGGGTC | SEQ ID N92 |
| LAMP1 | GGGTCACGTGCCGC | SEQ ID N93 |
| LAMP1 | GGGTCACGTGCCGC | SEQ ID N94 |
| LAMP1 | GGGTCACGTGTCGG | SEQ ID N95 |
| LAMP1 | GGATCACGTGACGC | SEQ ID N96 |
| LAMP1 | AGCTCACGTGACAA | SEQ ID N97 |
| M6PR | TGGTCACATGCCGC | SEQ ID N98 |
| MANBA | CGCTCAGCTGACCT | SEQ ID N99 |
| MCOLN1 | CGGTCACGTGAGGG | SEQ ID N100 |
| MCOLN1 | GGGTCACGTGACCG | SEQ ID N101 |
| MCOLN1 | AGATCAGCTGATGC | SEQ ID N102 |
| MFSD8 | CCGTCAGGTGCGCA | SEQ ID N103 |
| NAGLU | CGGTCACGAGACGC | SEQ ID N104 |
| NAGPA | GAGTCACGTGAGGC | SEQ ID N105 |
| NAGPA | AGCCCACATGACCC | SEQ ID N106 |
| NEU1 | GGGTCACGCGCTCC | SEQ ID N107 |
| NEU1 | GGGTCAGCTGACTC | SEQ ID N108 |
| P76 | CAGTCACGTGACCC | SEQ ID N109 |
| PLD3 | CCATCACGTGACCG | SEQ ID N110 |
| PLD3 | CCGTCACGTGACCG | SEQ ID N111 |
| PPT1 | CGGTCATGTGACAC | SEQ ID N112 |
| PSAP | GGATCAGCTGACGC | SEQ ID N113 |
| PSEN1 | AAATCAAGTGACCC | SEQ ID N114 |
| PSEN1 | CCGTCAGGTGGGGA | SEQ ID N115 |
| PSEN2 | CCCGCACGTGAGGG | SEQ ID N116 |
| RNASE6 | GCCTCAGATGAGGC | SEQ ID N117 |
| RNASET2 | GGGGCAGGTGAGGC | SEQ ID N118 |
| SCPEP1 | CCGTCACGTGATGG | SEQ ID N119 |
| SGSH | CGCGCACGTGACCG | SEQ ID N120 |
| SLC36A1 | GGAGCACGTGACCT | SEQ ID N121 |
| SLC36A1 | GGATCACGTGATGA | SEQ ID N122 |
| SUMF1 | CAGCCACGTGACCA | SEQ ID N123 |
| SUMF1 | GGGTCACATGGCCC | SEQ ID N124 |
| TMEM55B | GAAACACGTGACCA | SEQ ID N125 |
| TMEM55B | AGGTCATGTGACAG | SEQ ID N126 |
| TOM1 | GCGTCACGTGACGG | SEQ ID N127 |
| TPP1 | AGCTCATGTGATCC | SEQ ID N128 |
| TPP1 | CCGTCACATGACAG | SEQ ID N129 |
| UVRAG | TAATCAGGTGACTC | SEQ ID N130 |
| VPS11 | CGACCACGTGATTC | SEQ ID N131 |
| VPS11 | AGCTCACGTGACAA | SEQ ID N132 |
| VPS18 | GAGTCAGCTGAGCT | SEQ ID N133 |
| VPS18 | GGATCACCTGGCAC | SEQ ID N134 |
| ZMYM6 | ACACCACGTGACGA | SEQ ID N135 |

A genome-wide analysis of human gene promoters showed that this palindrome is preferentially associated with genes belonging to degradative pathways, including the following Gene Ontology (GO) categories: ubiquitin cycle; endocytosis; macromolecule catabolism; hydrolases. However, the most significantly enriched GO category was 'Lysosomal biogenesis and function'. Therefore authors named this sequence as 'Coordinated Lysosomal Expression And Regulation" (CLEAR) element.

A luciferase assay showed that the CLEAR element promotes gene transcription (Figure 2). A DNA decoy assay showed that the CLEAR element exerts a tight and essential control to the expression of lysosomal genes (Figure 3). Such experiments were repeated by using different CLEAR elements, in different combinations, and a molecule comprising four repeats each having the consensus sequence of SEQ ID No. 3. Results were confirmed.

Sequences similar to the CLEAR consensus are bound in vitro by members of the MiT family of b-HLH transcription factors [1-3]. The MiT family is composed of four members in humans: MITF, TFE3, TFEB, and TFEC. Authors performed transient expression of MiT proteins in cultured cells (HeLa) by transfection of an expression vector (pcDNA3.1, Invitrogen) carrying the human cDNA for MITF, TFE3, TFEB or TFEC. Twenty-four hours post-transfection, authors monitored the expression of lysosomal genes and found a general increase in the expression of lysosomal genes in cells transfected with TFEB, but not with MITF, TFE3, or TFEC; only CLEAR genes were responsive to TFEB (Figure 4). Conversely, monitoring of a random pool of genes unrelated to the degradative function showed no significant variation in their expression (Figure 4).

RNA interference experiments showed that silencing of endogenous TFEB leads to the downregulation of CLEAR genes (Figure 5) and massive cell death.

To demonstrate that TFEB regulates lysosomal genes by direct binding to CLEAR sites at their promoters, authors performed chromatin immunoprecipitation assay (ChIP). HeLa cell lines carrying a tagged version of TFEB (3xFLAG-TFEB) were established and CHIP was performed by using an anti-FLAG antibody.

Immunoprecipitated chromatin was analyzed by real-time qPCR. The analysis showed that lysosomal promoters carrying CLEAR sites were highly enriched in the immunoprecipitated fraction compared to control sites, demonstrating a direct interaction between TFEB and CLEAR sites (Figure 6).

To define the transcriptional targets of TFEB on a genome-wide scale authors performed microarray analysis of the HeLa transcriptome after TFEB transient over-expression. Gene Set Enrichment Analysis (GSEA) performed on genes ranked accordingly to their fold-induction showed that genes with CLEAR sites tend to be up-regulated after TFEB over-expression (Enrichment Score = 0.84; P < 0.0001) (Figure 7), confirming that TFEB specifically regulates genes with CLEAR sites at their promoters. A statistical analysis of transcriptional changes showed that 224 genes were up-regulated, and 6 down-regulated, at a false discovery rate < 0.05 following TFEB over-expression. Up-regulated genes were greatly enriched with lysosomal genes and other functional classes related to the lysosomal biogenesis and function (Figure 8).

Taken together, the analysis demonstrates that TFEB is a master transcriptional modulator of the lysosomal/degradative system by direct binding to the promoters of lysosomal/degradative genes.

To evaluate the impact of TFEB over-expression on the lysosomal function, authors performed enzymatic assays on lysosomal hydrolases cathepsin D (CD) and beta-glucosidase. Protein extracts were prepared from HeLa cells stably overexpressing a transgene carrying the cDNA of native TFEB. The results showed an increase in the enzymatic activities of both hydrolases (Figure 9), demonstrating that TFEB is able to enhance lysosomal activity.

The lysosomal system, and cathepsin D in particular, are involved in the clearance of pathogenic peptides such as Huntingtin, α-synuclein and amyloid β [6-12], the accumulation on which cause Huntington's disease, Parkinson's disease, and

Alzheimer's disease, respectively. Having determined that TFEB enhances CD expression and activity, authors tested its capability to promote the clearance of pathogenic peptides. To this aim, they used the rat neuronal cell model HD43 carrying an inducible transgene for the expanded, pathogenic Huntingtin isoform HD43(Q105) [13]. After induction of the HD43(Q105) transgene, HD43 cells were electroporated with a TFEB-3xFLAG vector or an empty vector. The accumulation of Huntingtin was then monitored at 12 and 24 hours post-transfection. Western blot analyses showed an increase of CD and a decrease of Huntingtin accumulation in cells transfected with TFEB-3xFLAG compared to control (Figure 10). Immunofluorescence analyses showed that the cells expressing TFEB-3xFLAG had little or no signal for Huntingtin staining (Figure 11). These results indicate that TFEB is able to induce the clearance of cytotoxic HD43(Q105).

In conclusion, authors found that most lysosomal genes form a regulatory gene network, and identified the key elements by which the cell controls the lysosomal gene network: a cis-regulatory motif, named CLEAR element, and its cognate transcription factor TFEB. Data show that TFEB can act as a master regulator of degradative pathways by enhancing the lysosomal system. The modulation of the CLEAR (Coordinated Lysosomal Expression And Regulation) network represents a valuable instrument to modulate cellular degradative pathways and has therapeutic application for the treatment of diseases caused by pathogenic intracellular storage, including neurodegenerative diseases such as Alzheimer's, Parkinson's, and Huntington's, as well as lysosomal storage disorders.

### Bibliography

1. Esumi, N., Kachi, S., Campochiaro, P. A. & Zack, D. J. VMD2 promoter requires two proximal E-box sites for its activity in vivo and is regulated by the MITF-TFE family. J Biol Chem 282, 1838-50 (2007).
2. Meadows, N. A. et al. The expression of Clcn7 and Ostm1 in osteoclasts is coregulated by microphthalmia transcription factor. J Biol Chem 282, 1891-904 (2007).
3. Steingrimsson, E., Copeland, N. G. & Jenkins, N. A. Melanocytes and the microphthalmia transcription factor network. Annu Rev Genet 38, 365-411 (2004).
4. Steingrimsson, E. et al. Mitf and Tfe3, two members of the Mitf-Tfe family of bHLH-Zip transcription factors, have important but functionally redundant roles in osteoclast development. Proc Natl Acad Sci U S A 99, 4477-82 (2002).
5. Steingrimsson, E., Tessarollo, L., Reid, S. W., Jenkins, N. A. & Copeland, N. G. The bHLH-Zip transcription factor Tfeb is essential for placental vascularization. Development 125, 4607-16 (1998).
6. Webb, J. L., Ravikumar, B., Atkins, J., Skepper, J. N. & Rubinsztein, D. C. Alpha-Synuclein is degraded by both autophagy and the proteasome. J Biol Chem 278, 25009-13 (2003).
7. Shin, Y., Klucken, J., Patterson, C., Hyman, B. T. & McLean, P. J. The co-chaperone carboxyl terminus of Hsp70-interacting protein (CHIP) mediates alpha-synuclein degradation decisions between proteasomal and lysosomal pathways. J Biol Chem 280, 23727-34 (2005).
8. Cuervo, A. M., Stefanis, L., Fredenburg, R., Lansbury, P. T. & Sulzer, D. Impaired degradation of mutant alpha-synuclein by chaperone-mediated autophagy. Science 305, 1292-5 (2004).
9. Kim, Y. J. et al. Lysosomal proteases are involved in generation of N-terminal huntingtin fragments. Neurobiol Dis 22, 346-56 (2006).
10. Qin, Z. H. et al. Autophagy regulates the processing of amino terminal huntingtin fragments. Hum Mol Genet 12, 3231-44 (2003).
11. Cataldo, A. M. et al. Gene expression and cellular content of cathepsin D in Alzheimer's disease brain: evidence for early up-regulation of the endosomal-lysosomal system. Neuron 14, 671-80 (1995).
12. Callahan, L. M., Vaules, W. A. & Coleman, P. D. Quantitative decrease in synaptophysin message expression and increase in cathepsin D message expression in Alzheimer disease neurons containing neurofibrillary tangles. J Neuropathol Exp Neurol 58, 275-87 (1999).
13. Sipione, S. et al. Early transcriptional profiles in huntingtin-inducible striatal cells by microarray analyses. Hum Mol Genet 11, 1953-65 (2002).

## Claims

1. A molecule able to modulate the expression of at least a gene involved in degradative pathways so to enhance the cellular degradative pathways and prevent or antagonize the accumulation of toxic compounds in a cell wherein said molecule acts on a CLEAR element, said element comprising at least one repeat of a nucleotide sequence having SEQ ID N. 3 as consensus sequence; and/or on the TFEB protein activity or amount.

2. The molecule according to claim 1 wherein said molecule acts on a CLEAR element comprising at least one repeat of a nucleotide sequence selected from the group from SEQ ID N. 3 to SEQ ID N. 135.

3. The molecule according to claim 1 or 2 wherein said gene involved in degradative pathways is a gene expressing a lysosomal protein.

4. The molecule according to claim 1 to 3 being a peptide.

5. The molecule according to claim 4 being the TFEB protein or a functional fragment thereof or a mutein thereof, that is able to enhance the expression of a gene involved in degradative pathways.

6. The molecule according to claim 1 to 3 being a microRNA or microRNA inhibitor.

7. The molecule according to any of previous claim for medical use.

8. The molecule according to claim 7 for neurodegenerative disorders' treatments.

9. The molecule according to claim 8 wherein the neurodegenerative disorder belongs to the group of Alzheimer, Parkinson and Huntington diseases.

10. The molecule according to claim 7 for lysosomal storage disorders' treatments.

11. A nucleic acid containing a coding sequence encoding for the molecule according to any of claims 1 to 6.

12. A vector for gene therapy comprising under appropriate regulative sequence the nucleic acid according to claim 11.
